(19) Europäisches Patentamt
European Patent Office
Office européen des brevets

(11) Veröffentlichungsnummer: **0 340 709**
**A1**

(12) **EUROPÄISCHE PATENTANMELDUNG**

(21) Anmeldenummer: **89107885.9**

(22) Anmeldetag: **29.04.89**

(51) Int. Cl.4: **C07C 87/28 , C07C 87/29 , C07C 103/375 , C07C 103/76 , C07C 103/58 , C07C 143/78 , C07C 127/17 , C07C 127/19 , C07C 125/063 , C07C 121/42 , C07C 101/04**

(30) Priorität: **04.05.88 DE 3815046**

(43) Veröffentlichungstag der Anmeldung:
**08.11.89 Patentblatt 89/45**

(84) Benannte Vertragsstaaten:
**DE FR GB IT**

(71) Anmelder: **BASF Aktiengesellschaft**
**Carl-Bosch-Strasse 38**
**D-6700 Ludwigshafen(DE)**

(72) Erfinder: **Hagen, Helmut, Dr.**
**Max-Slevogt-Strasse 17 e**
**D-6710 Frankenthal(DE)**
Erfinder: **Pfister, Juergen, Dr.**
**Ziegelofenweg 1**
**D-6720 Speyer(DE)**
Erfinder: **Wuerzer, Bruno, Dr.**
**Ruedigerstrasse 13**
**D-6701 Otterstadt(DE)**
Erfinder: **Meyer, Norbert, Dr.**
**Dossenheimer Weg 22**
**D-6802 Ladenburg(DE)**

(54) **(3-Chlor-2-methylhenyl)ethylamine, Verfahren zur Herstellung und ihre Verwendung.**

(57) (3-Chlor-2-methylphenyl)ethylamine der allgemeinen Formel I

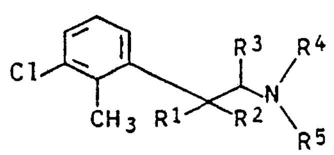

I

in der die Substituenten folgende Bedeutung haben:
$R^1$, $R^4$ Wasserstoff, $C_1$-$C_8$-Alkyl, ggf. substituiertes Arylmethyl oder Hetarylmethyl,
$R^2$ Wasserstoff oder gemeinsam mit $R^1$ $=CHR^6$ worin
$R^6$ ggf. substituiertes Aryl oder Hetaryl bedeutet,
$R^3$ Wasserstoff, $C_1$-$C_6$-Alkyl oder ggf. substituiertes Aryl,
$R^5$ eine Gruppe $R^1$, ggf. substituiertes $C_2$-$C_3$-Alkyl,
-C(X)Y, worin
X Sauerstoff- oder Schwefel und
Y ggf. substituiertes $C_1$-$C_4$-Alkyl,
$C_1$-$C_4$-Alkoxy, Aryloxy, Arylamino, $C_1$-$C_4$-Alkylarylamino- oder Diarylaminogruppe, $C_1$-$C_4$-Alkoxycarbonyl-$C_1$-$C_2$-alkylamino, Amino, $C_1$-$C_4$-Alkylamino, Di-$C_1$-$C_4$-Alkylamino, $C_1$-$C_4$-Alkoxy oder ein Rest $R^1$,

EP 0 340 709 A1

bedeutet,
$-SO_2R^7$ worin $R^7$ Amino, $C_1$-$C_4$-Alkylamino, Di-$C_1$-$C_4$-Alkylamino, $C_1$-$C_4$-Alkoxy oder ein Rest $R^1$ bedeutet oder
$-P(X)R^8R^9$ worin X die vorstehend gegebene Bedeutung hat und
$R^8$, $R^9$ $C_1$-$C_4$-Alkoxy, $C_1$-$C_4$-Alkylamino, Di-$C_1$-$C_4$-Alkylamino, Aryloxy und/oder $C_1$-$C_4$-Halogenalkoxy bedeuten, oder
$R^4$, $R^5$ gemeinsam $=CHR^6$ oder

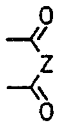 ,

worin
Z $C_2$-$C_4$-Methylenbrücke, ggf. substituiertes Ethylen oder ggf. substituiertes Ethenylen,
bedeutet,
wobei $R^3$ Wasserstoff bedeutet, wenn $R^2$ nicht Wasserstoff bedeutet, sowie deren umweltverträgliche Salze, Verfahren zur Herstellung und Verwendung.

# (3-Chlor-2-methylhenyl)ethylamine, Verfahren zur Herstellung und ihre Verwendung

(3-Chlor-2-methylphenyl)ethylamine der allgemeinen Formel I

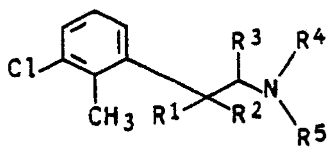

I

in der die Substituenten folgende Bedeutung haben:

$R^1$ Wasserstoff, eine $C_1$-$C_8$-Alkylgruppe, eine Arylmethylgruppe oder eine Hetarylmethylgruppe, wobei die aromatischen Reste ein bis fünf Halogenatome und/oder ein bis drei der folgenden Substituenten tragen können: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio und/oder $C_1$-$C_4$-Halogenalkylthio,

$R^2$ Wasserstoff oder gemeinsam mit $R^1$ eine Gruppe $=CHR^6$ worin

$R^6$ eine Arylgruppe oder eine Hetarylgruppe bedeutet, wobei diese aromatischen Reste ein bis fünf Halogenatome und/oder ein bis drei der folgenden Substituenten tragen können: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio und/oder $C_1$-$C_4$-Halogenalkylthio,

$R^3$ Wasserstoff, eine $C_1$-$C_6$-Alkylgruppe oder ein Arylrest, wobei der aromatische Rest ein bis fünf Halogenatome und/oder ein bis drei der folgenden Gruppen tragen kann: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio und/oder $C_1$-$C_4$-Halogenalkylthio,

$R^4$ eine der unter $R^1$ genannten Gruppen,

$R^5$ eine der unter $R^1$ genannten Gruppen,

eine $C_2$-$C_3$-Alkylgruppe, welche einen der folgenden Substituenten trägt: Nitro, Cyano, $-COR^7$oder $SO_2R^7$ worin

$R^7$ eine Aminogruppe, eine $C_1$-$C_4$-Alkylaminogruppe, eine Di-$C_1$-$C_4$-Alkylaminogruppe, eine $C_1$-$C_4$-Alkoxygruppe oder eine der unter $R^1$ genannten Gruppen

bedeutet,

ein Rest -C(X)Y, worin

X ein Sauerstoff- oder ein Schwefelatom und

Y eine $C_1$-$C_4$-Alkylgruppe, welche einen der folgenden Reste tragen kann: Amino, $C_1$-$C_4$-Alkylamino, Di-$C_1$-$C_4$-Alkylamino, $C_1$-$C_4$-Alkoxy, Carboxyl oder $C_1$-$C_4$-Alkoxycarbonyl,

eine $C_3$-$C_6$-Alkenylgruppe, welche eine Aminogruppe tragen kann, wobei diese Aminogruppe ihrerseits eine $C_1$-$C_4$-Alkoxycarbonyl-$C_1$-$C_2$-alkylgruppe und/oder ein bis zwei der unter $R^3$ genannten Gruppen tragen kann:

eine $C_1$-$C_4$-Alkoxygruppe, eine Aryloxygruppe, eine Arylaminogruppe, eine $C_1$-$C_4$-Alkylarylaminogruppe oder eine Diarylaminogruppe, eine $C_1$-$C_4$-Alkoxycarbonyl-$C_1$-$C_2$-alkylaminogruppe oder eine der unter $R^7$ genannten Gruppen

ein Rest $-SO_2R^7$ worin $R^7$ die vorstehend gegebene Bedeutung hat oder

ein Rest $-P(X)R^8R^9$ worin X die vorstehend gegebene Bedeutung hat und

$R^8$, $R^9$ $C_1$-$C_4$-Alkoxy, $C_1$-$C_4$-Alkylamino, Di-$C_1$-$C_4$-Alkylamino, Aryloxy und/oder $C_1$-$C_4$-Halogenalkoxy bedeuten, oder

$R^4$,$R^5$ gemeinsam einen Rest $=CHR^6$ wie vorstehend definiert, oder gemeinsam einen Rest

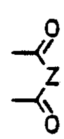

,

worin

Z eine $C_2$-$C_4$-Methylenbrücke,

eine Ethylenbrücke, welche ihrerseits Teil eines 5- bis 6-gliedrigen Cycloalkylrestes ist, wobei dieser Rest ein bis vier Halogenatome und/oder $C_1$-$C_4$-Alkylgruppen tragen kann,

eine Ethenylenbrücke, welche Teil eines 5- bis 8-gliedrigen Cycloalkenylrings oder einen 5- bis 6-gliedrigen

aromatischen oder heteroaromatischen Ringes sein kann, wobei diese Reste ihrerseits ein bis vier Halogenatome und/oder ein bis zwei der folgenden Gruppen tragen können: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Akylthio und/oder $C_1$-$C_4$-Halogenalkylthio
bedeutet,
wobei $R^3$ Wasserstoff bedeutet, wenn $R^2$ nicht Wasserstoff bedeutet, sowie deren umweltverträgliche Salze.

Außerdem betrifft die Erfindung die Herstellung der Verbindungen I, ihre Verwendung als Herbizide, sowie Herbizide, die die Verbindungen I enthalten und ein Verfahren zur Bekämpfung unerwünschten Pflanzenwuchses.

Aus der GB-PS 760,968 sind 3-Chlor-2-methylbenzylcarbonsäurederivate der Formel XII

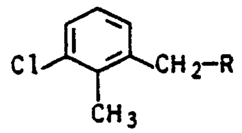

XII

als Wachstumsregulatoren und Herbizide bekannt, wobei der Rest R dort eine Carbonsäure-, eine Carbonsäureamid- oder eine Nitrilfunktion bedeutet.

Es werden jedoch Verbindungen mit günstigeren Eigenschaften als Herbizide gesucht.

Entsprechend dieser Aufgabe wurden die eingangs definierten (3-Chlor-2-methylphenyl)ethylamine I gefunden.

Außerdem wurden Verfahren zur Herstellung der Verbindungen I und ihre Verwendung als Herbizide gefunden.

Man erhält die Verbindungen I auf verschiedenen Reaktionswegen, welche in analoger Weise aus der Literatur bekannt sind. Beispielhaft sei im folgenden auf sieben Synthesemöglichkeiten (A bis G) näher eingegangen:

A) Man erhält die Verbindungen I in denen $R^2$, $R^3$, $R^4$ und $R^5$ Wasserstoff bedeuten, beispielsweise dadurch, daß man ein entsprechendes Benzylhalogenid II in einem inerten aprotischen organischen Lösungsmittel in Gegenwart eines Katalysators mit einem Alkalimetall- oder Erdalkalimetalolcyanid zum entsprechenden Benzylcyanid III umsetzt und dieses anschließend in an sich bekannter Weise zum (3-Chlor-2-methylphenyl)ethylamin I ($R^2 = R^3 = R^4 = R^5 = H$) reduziert.

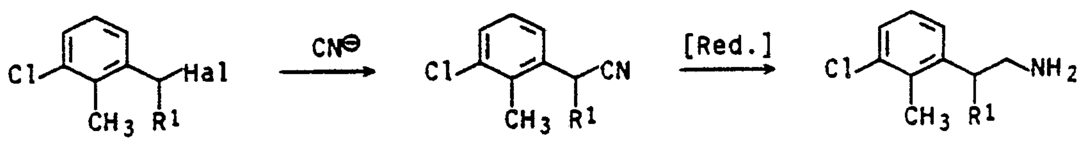


II III Ia

Hal in Formel II bedeutet dabei ein Halogenatom wie Fluor, Chlor, Brom und Iod, vorzugsweise Chlor und Brom.

Geeignete Alkalimetall- bzw. Erdalkalimetallcyanide sind Natriumcyanid und Kaliumcyanid.

Die Umsetzung von II zu III wird im allgemeinen in einem polaren organischen Lösungsmittel wie Dimethylsulfoxid, Dimethylformamid und N-Methylpyrrolidon bei Temperaturen von 20 bis 150 °C, vorzugsweise 80 bis 120 °C durchgeführt.

Geeignete Katalysatoren sind beispielsweise Kaliumiodid und Tetrabutylammonumiodid.

Die anschließende Reduktion des Benzylcyanids III zum Phenylethylamin Ia erfolgt nach einer der in Houben-Weyl, B. 11/1, S. 545 ff angegebenen Methoden.

Bevorzugt wird die katalytische Hydrierung mit Raney-Nickel in Gegenwart von Ammoniak. Die Umsetzung kann bei atmosphärischem Druck bei Raumtemperatur, unter Druck bei Raumtemperatur oder erhöhter Temperatur durchgeführt werden. Beim Arbeiten unter Druck kann Ammoniak ganz oder teilweise die Rolle des Lösungsmittels übernehmen. Bevorzugt sind Reaktionsbedingungen, bei denen Ammoniak als Lösungsmittel, eine

Temperatur zwischen 30 und 150 °C, bevorzugt 70 bis 90 °C und ein Druck von 10 bis 100 bar, bevorzugt etwa 50 bar angewendet werden.

Oder man verwendet als Reduktionsmittel ein komplexes Hydrid, bevorzugt Aluminiumhydrid und Lithiumaluminiumhydrid. Die Reaktion kann in diesem Fall in Gegenwart eines inerten Lösungsmittels, bevorzugt Tetrahydrofuran, Diethylether, Dimethoxyethan, bei einer Temperatur zwischen 0 °C und der

Rückflußtemperatur des betreffenden Lösungsmittels durchgeführt werden. Bevorzugt wird die Verwendung von Aluminiumhydrid als Reduktionsmittel, das in situ durch Reaktion von Lithiumaluminiumhydrid mit Schwefelsäure erzeugt werden kann.

B) Man erhält die Verbindungen, in denen $R^3$, $R^4$ und $R^5$ Wasserstoff bedeuten beispielsweise dadurch, daß man ein Benzylcyanid IIIa in dem $R^1$ Wasserstoff bedeutet, in an sich bekannter Weise in einem inerten organischen Lösungsmittel in Gegenwart einer Base mit einem Aldehyd der Formel IV kondensiert und das so erhaltene Vinylcyanid V anschließend in an sich bekannter Weise zum (3-Chlor-2-methylphenyl)-ethylamin Ib ($R^3 = R^4 = R^5 = H$) reduziert.

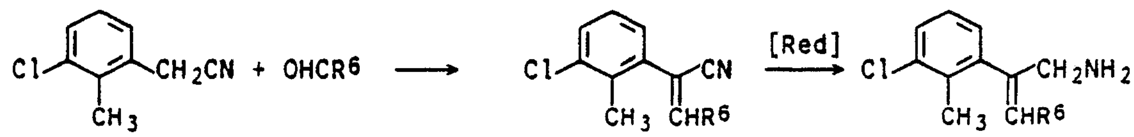


IIa IV V Ib

Die Umsetzung von IIIa mit IV wird im allgemeinen in einem Lösungsmittel wie Methanol, Ethanol, Benzol, Toluol und Chlorbenzol bei Temperaturen von 30 bis 180 °C, vorzugsweise 50 bis 80 °C durchgeführt.

Als Basen eignen sich insbesondere Natriummethylat und Kalium-t-butylat.

Es kann daneben von Vorteil sein, der Reaktionsmischung β-Alanin oder Piperidiniumacetat zuzumischen.

Die anschließende Reduktion von V zu Ib kann entsprechend den bei Punkt A geschilderten Bedingungen durchgeführt werden.

Insbesondere geeignet ist die katalytische Reduktion mit Raney-Nickel in inerten Lösungsmitteln wie beispielsweise Ethanol und Tetrahydrofuran bei Temperaturen von 10 bis 50 °C, vorzugsweise 15 bis 40 °C bei einem Wasserstoffdruck von 1 bis 50 bar, vorzugsweise 1,05 bis 10 bar.

C) Die Verbindungen I in denen $R^2$, $R^4$ und $R^5$ Wasserstoff bedeuten, erhält man beispielsweise dadurch, daß man ein entsprechendes Phenylcarbonylderivat VI in an sich bekannter Weise in einem inerten organischen Lösungsmittel in Gegenwart einer Base mit einem Nitromethanderivat VII zum Nitrostyrylderivat VIII kondensiert und dieses anschließend in üblicher Weise zum (3-Chlor-2-methylphenyl)ethyl Ic ($R^2 = R^4 = R^5 = H$) reduziert.

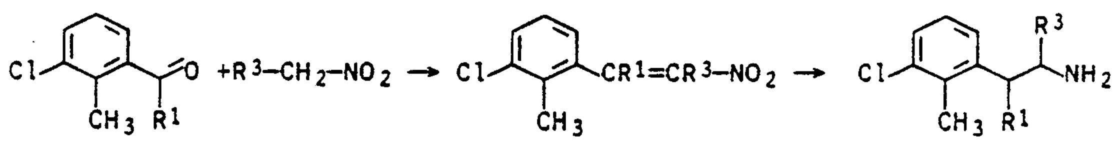

VI VII VIII Ic

Die Umsetzung von VI mit VII erfolgt nach den Standardreaktionsbedingungen für die Knoevenagel-Kondensation von Aldehyden mit CH-aciden Nitroalkanen, wie sie in Houben-Weyl Bd. 10/1 beschrieben sind.

Bevorzugt werden Reaktionsbedingungen bei denen als Lösungsmittel Alkohole, bevorzugt Methanol, Katalysatoren wie Alkalimetallhydroxide, Amine wie Butylamin, Diethylamin, Piperidin bevorzugt Ethylendiammoniumdiacetat und eine Temperatur zwischen 0 °C und der der Rückflußtemperatur des betreffenden Lösungsmittels, bevorzugt 20 bis 30 °C, verwendet werden.

Die so erhaltenen Nitrostyrylverbindungen VIII können entsprechend den bei Punkt A zitierten Methoden zu den Phenylethylaminen Ic reduziert werden.

Bevorzugt eignet sich hierbei die katalytische Hydrierung an Edelmetallkatalysatoren wie Platin, Palladium, Rhodium oder Nickel, gegebenenfalls auf einen Träger wie Aktivkohle, Kieselgel oder Aluminiumoxid, in einem Verdünnungsmittel wie $C_1$-$C_6$-Alkohol, einem Carbonsäureester dieser Alkohole, einer Carbonsäure wie Essigsäure oder Propionsäure, einem cyclischen Ether wie Dioxan oder Tetrahydrofuran, einem Amid wie Dimethylformamid oder N-Methylpyrrolidon oder einem geeigneten Gemisch.

Reaktionstemperaturen zwischen 20 und 100 °C und eventuell erhöhter Druck bis 100 bar eignen sich für diese Reaktion.

An verwandten ω-Nitrostyrolderivaten wird die Tauglichkeit der katalytischen Reduktionsverfahren zur Herstellung der entsprechenden Arylethylamine belegt:

- R.T. Coutts et al., Can. J. Chem. 52, 395 (1974).
- M.Freifelder, Practical Catalytic Hydrogenation, Wiley Intersci. Publ., New York, London, Sydney, Toronto 1971.

Des weiteren kann die Reduktion auch gemäß den in Houben-Weyl, Bd. 11/2 angegebenen Methoden durchgeführt werden.

Man verwendet dann als Reduktionsmittel komplexe Metallhydride, bevorzugt Lithiumaluminiumhydrid.

Die Reaktion wird in Gegenwart eines inerten Lösungsmittel, bevorzugt einem Ether wie Tetrahydrofuran, Diethylether, Dimethoxyethan durchgeführt, bei einer Temperatur zwischen 0° C und der Rückflußtemperatur des betreffenden Lösungsmittels.

D) Die Verbindungen I in denen $R^4$ und/oder $R^5$ nicht Wasserstoff bedeuten, erhält man beispielsweise dadurch, daß man ein (3-Chlor-2-methylphenyl)ethylamin Id in an sich bekannter Weise in einem inerten organischen Lösungsmittel in Gegenwart einer Base mit einem Halogenid der Formel IXa bzw. IXb derivatisiert.

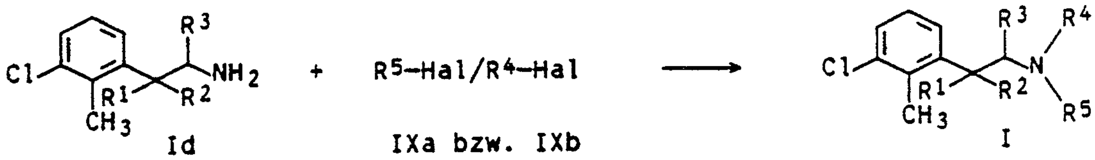


Id IXa bzw. IXb I

Hal in den Formeln IXa bzw. IXb bedeutet hier ein Halogenatom wie vorzugsweise Chlor, Brom und Iod.

Die Umsetzungen werden bevorzugt in Gegenwart einer inerten Hilfsbase z.B. Triethylamin oder Pyridin durchgeführt. Gegebenenfalls kann auch eine überschüssige Menge des Phenethylamins der Formel Ia als säurebindendes Mittel eingesetzt werden. Die Umsetzungen werden bei -10 °C bis +40 °C in einem Lösungsmittel wie einem chlorierten Kohlenwasserstoff, etwa Methylenchlorid, Tetrachlormethan oder einem Ether wie Tetrahydrofuran, Dioxan, Diethylether, Dimethoxyethan oder einem Aromaten wie Toluol oder Chlorbenzol oder Pyridin durchgeführt.

Einen allgemeinen Überblick über diese Reaktion liefert Houben-Weyl, Bd. 11/2, Houben-Weyl, Bd. 9/2, Houben-Weyl, Bd. 12/2.

E) Daneben erhält man die Verbindungen I, in denen $R^4$ und $R^5$ gemeinsam eine Gruppe -CO-Z-CO- bedeuten, dadurch, daß man ein entsprechendes (3-Chlor-2-methylphenyl)ethylamin Id in an sich bekannter Weise in einem inerten organischen Lösungsmittel mit einem Carbonsäureanhydrid der Formel X kondensiert.

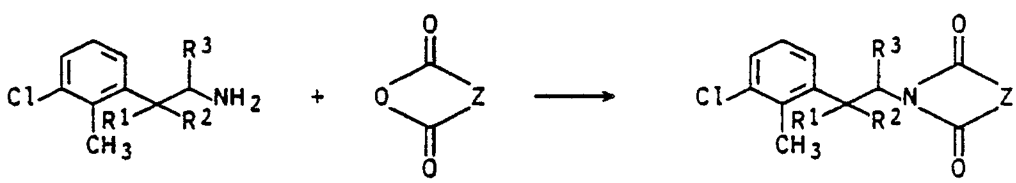

Id X I

Diese Umsetzung kann wie allgemein bekannt (Houben-Weyl Bd. 11/2, S. 16) ohne oder mit Lösungsmitteln durchgeführt werden. Bevorzugt sind Reaktionsbedingungen, bei denen Eisessig als Reaktionsmedium und eine Temperatur zwischen 20 und 115 °C, bevorzugt Rückflußtemperatur verwendet werden.

F) Man erhält die Verbindungen I, in denen $R^4$ und $R^5$ gemeinsam eine Gruppe $=CHR^6$ bedeuten, beispielsweise dadurch, daß man ein entsprechendes (3-Chlor-2-methylphenyl)ethylamin Id in an sich bekannter Weise in einem inerten organischen Lösungsmittel in Gegenwart einer Base mit einem Aldehyd der Formel IV kondensiert.

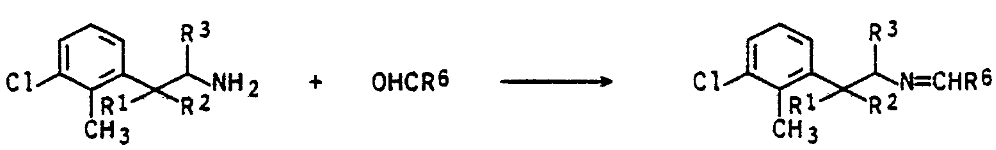

Id IV I

Diese Umsetzung kann wie allgemein bekannt (Houben-Weyl, Bd. 11/2, S. 74). ohne oder mit Lösungsmittel durchgeführt und mit sauren oder basischen Katalysatoren beschleunigt werden. Vorteilhaft wirkt sich die Entfernung des Reaktionswassers durch azeotrope Destillation aus. Die Umsetzungen werden bevorzugt in Toluol als Lösungsmittel bei Rückflußtemperatur am Wasserabscheider durchgeführt.

Die Phenethylaminderivate 1c in denen $R^2$ Wasserstoff bedeutet, können durch Anwendung der Leuckert-Wallach-Reaktion, d.h. durch Umsetzung des Phenethylamins der Formel Ia oder Ic unter Verwendung von Aldehyden und Ketonen als Alkylierungsmittel und Ameisensäure als Reduktionsmittel erhalten werden. Die Durchführung erfolgt in Anlehnung an die in Houben-Weyl, Bd. 11/1, S. 648-664 aufgeführten Standardmethoden. Bevorzugt ist eine Reaktionszeit um 15 Stunden bei Rückflußtemperatur.

G) Verfahren zur Herstellung der Verbindungen I gemäß Anspruch 1, in denen $R^4$ und/oder $R^5$ eine Gruppe -C(X)Y bedeuten, dadurch gekennzeichnet, daß man ein entsprechendes (3-Chlor-2-methylphenyl)-ethylamin Id gemäß Anspruch 5 in an sich bekannter Weise in einem inerten organischen Lösungsmittel in Gegenwart einer Base an ein Alken der Formel XI addiert.

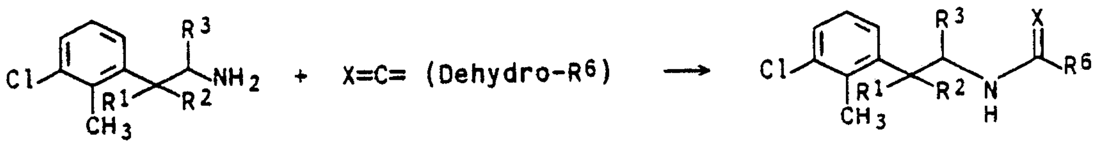


Id XI

Unter Dehydro-$R^6$ in Formel XI ist eine Gruppe $R^6$ zu verstehen, welche am Bindungsatom dehydriert ist, beispielsweise =NH, =N- ($C_1$- bis $C_4$-Alkyl) oder =CH-($C_1$- bis $C_7$-Alkyl) u.a.

Die Umsetzung von Id mit XI wird im allgemeinen in einem Lösungsmittel wie Benzol, Toluol, Xylol und Chlorbenzol bei Temperaturen von 20 bis 180 °C, vorzugsweise 50 bis 80 °C durchgeführt.

Als Basen eignen sich beispielsweise tertiäre Amine und Alkoholate, insbesondere Triethylamin und Natriummethylat.

Es kann zusätzlich von Vorteil sein der Reaktionsmischung Kupferacetat zuzusetzen.

Im hinblick auf die biologische Wirksamkeit sind Verbindungen I bevorzugt, in denen die Substituenten folgende Bedeutung haben:

$R^1$ Wasserstoff,

$C_1$-$C_8$-Alkyl wie Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, insbesondere Methyl,

Arylmethyl wie Benzyl und Naphthylmethyl, insbesondere Benzyl,

Hetarylmethyl wie Pyr-1-olylmethyl, Pyrazolylmethyl, Imidazolylmethyl, Isoxazolylmethyl, Oxazolylmethyl, Isothiazolylmethyl, Thiazolylmethyl, Furanylmethyl, Thienylmethyl, Pyrylmethyl, Pyrimidylmethyl, Pyrazinylmethyl, Pyridazinylmethyl, Chinolinylmethyl und Isochinolinylmethyl,

wobei die aromatischen und heteroaromatischen Reste ein bis fünf, insbesondere ein bis zwei Halogenatome wie Fluor, Chlor, Brom und Iod, insbesondere Fluor, Chlor und Brom,

und/oder ein bis drei der folgenden Substituenten tragen können

Alkyl wie Methyl, Ethyl, Propyl, 1-Methylethyl, Butyl, 1-Methylpropyl, 2-Methylpropyl, 1,1-Dimethylethyl, insbesondere Methyl,

Halogenalkyl wie Fluormethyl, Difluormethyl, Trifluormethyl, Chlordifluormethyl, Dichlorfluormethyl, Trichlormethyl, 1-Fluorethyl, 2-Fluorethyl, 2,2-Difluorethyl, 2,2,2-Trifluorethyl, 2-Chlor-2,2-difluorethyl, 2,2-Dichlor-2-fluorethyl, 2,2,2-Trichlorethyl und Pentafluorethyl, insbesondere Triflouormethyl,

Alkoxy wie Methoxy, Ethoxy, Propoxy, 1-Methylethoxy, Butoxy, 1-Methylpropoxy, 2-Methylpropoxy und 1,1-Dimethylethoxy, insbesondere Methoxy und Ethoxy,

Halogenalkoxy wie Difluormethoxy, Trifluormethoxy, Chlor-difluormethoxy, Dichlorfluormethoxy, 1-Fluorethoxy, 2-Fluorethoxy, 2,2-Difluorethoxy, 1,1,2,2-Tetrafluorethoxy, 2,2,2-Trifluorethoxy, 2-Chlor-1,1,2-trifluorethoxy und Pentafluorethoxy, insbesondere Diflormethoxy und Trifluormethoxy,

Alkylthio wie Methylthio, Ethylthio, Propylthio, 1-Methylethylthio, Butylthio, 1-Methylpropylthio, 2-Methylpropylthio und 1,1-Dimethylethylthio, insbesondere Methylthio,

Halogenalkylthio wie Difluormethylthio, Trifluormethylthio, Chlordifluormethylthio, Dichlorfluormethylthio, 1-Fluorethylthio, 2-Fluorethylthio, 2,2-Difluorethylthio, 1,1,2,2-Tetrafluorethylthio, 2,2,2-Trifluorethylthio, 2-Chlor-1,1,2-trifluorethylthio und Pentafluorethylthio,

$R^2$ Wasserstoff oder

gemeinsam mit $R^1$ eine Gruppe =$CHR^6$ worin

$R^6$ Aryl und Hetaryl wie Phenyl, Naphthyl, Pyrrolyl, Pyrazolyl, Imidazolyl, Isoxazolyl, Oxazolyl, Isothiazolyl, Thiazolyl, Furanyl, Thienyl, Pyridyl, Pyrimidyl, Pyrazinyl, Pyridazinyl, Chinolinyl einer Isochinolinyl, insbesondere Phenyl bedeutet,

wobei diese Ringe im allgemeinen und im besonderen die bei $R^1$ genannten folgenden Substituenten tragen können: Halogen, Alkyl, Halogenalkyl, Alkoxy, Halogenalkoxy, Alkylthio und/oder Halogenalkylthio,

$R^3$ Wasserstoff,

Alkyl und Aryl im allgemeinen und im besonderen wie unter $R^1$ genannt, wobei die aromatischen Reste ebenfalls substituiert sein können,

$R^4$ im allgemeinen oder im besonderen eine der unter $R^1$ genannten Gruppen,

$R^5$ eine der unter $R^1$ genannten Gruppen, insbesondere Ethyl oder Propyl, welches einen der folgenden Substituenten trägt: Nitro, Cyano, -$COR^7$ oder -$SO_2R^7$, wobei

$R^7$ Amino,

Alkylamino wie Methylamino, Ethylamino, Propylamino, 1-Methylethylamino, Butylamino, 1-Methylpropylamino, 2-Methylpropylamino und 1,1-Dimethylethylamino, insbesondere Methylamino,

Dialkylamino wie Dimethylamino, Diethylamino, Dipropylamino, Di-1-methylethylamino, Dibutylamino, Di-1-methylpropylamno, Di-2-methylpropylamino, Di-1,1-Dimethylethylamino, insbesondere Dimethylamino,

Alkoxy im allgemeinen und im besonderen wie unter $R^1$ genannt,

oder eine der unter $R^1$ genannten Gruppen,

bedeutet,

ein Rest -C(X)Y, worin

X Sauerstoff oder Schwefel und

Y - Alkyl wie Methyl, Ethyl, Propyl, 1-Methylethyl, Butyl, 1-Methylpropyl, 2-Methylpropyl und 1,1-Dimethylethyl, welches einen der folgenden Reste tragen kann:

Amino, Alkylamino wie unter $R^7$ genannt, Dialkylamino wie unter $R^7$ genannt, Alkoxy wie unter $R^1$ genannt, Carbonyl oder Alkoxycarbonyl wie Methoxycarbonyl, Ethoxycarbonyl, Propyloxycarbonyl, 1-Methylethoxycarbonyl, Butyloxycarbonyl und 1,1-Dimethyl-ethoxycarbonyl,

- Alkenyl wie 2-Propenyl, 2-Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1-Methyl-3-butnyl, 2-Methyl-3-butenyl, 3-Methyl-3-butenyl, 1,1-Dimethyl-2-propenyl, 1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyi, 1-Mthyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-2-butenyl, 1,2-Dimethyl-3-butenyl, 1,3-Dimethyl-2-butenyl, 1,3-Dimethyl-3-butenyl, 2,2-Dimethyl-3-butenyl, 2,3-Dimethyl-2-butenyl, 2,3-Dimethyl-3-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl und 1-Ethyl-2-methyl-2-propenyl,

welches eine Aminogruppe tragen kann, wobei die Aminogruppe ihrerseits einfach durch

Alkoxycarbonylalkyl wie Methoxycarbonylmethyl, Ethoxycarbonylmethyl, Propyloxycarbonylmethyl, 1-Methylethoxycarbonmethyl, Butyloxycarbonylmethyl, 1-Methylpropylcarbonylmethyl, 2-Methylpropylcarbonylmethyl, 1,1-Dimethylethoxycarbonylmethyl,

1-(Methoxycarbonyl)ethyl, 1-(Ethoxycarbonyl)ethyl, 1-(Propyloxycarbonyl)ethyl, 1-(1-Methylethoxycarbonyl)-ethyl, 1-(Butyloxycarbonyl)ethyl, 1-(1-Methylpropylcarbonyl)ethyl, 1-(2-Methylpropylcarbonyl)ethyl und 1-(1,1-Dimethylethoxycarbonyl)ethyl,

2-(Methoxycarbonyl)ethyl, 2-(Ethoxycarbonyl)ethyl, 2-(Propyloxycarbonyl)ethyl, 2-(1-Methylethoxycarobnyl)-ethyl, 2-(Butyloxycarbonyl)ethyl, 2-(1-Methylpropylcarbonyl)ethyl, 2-(2-Methylpropylcarbonyl)ethyl und 2-(1,1-Dimethylethoxycarbonyl)ethyl,

und/oder ein- bis zweifach durch die unter $R^3$ genannten Gruppen substituiert sein kann,

- Alkoxy wie unter $R^1$ genannt,

- Aryloxy wie Phenoxy und Naphthyloxy,

- oder eine der unter $R^7$ genannten Gruppen,

ein Rest -$SO_2R^7$, worin $R^7$ die vorstehend gegebene Bedeutung hat,

ein Rest -$P(Y)R^8R^9$, worin X die vorstehend gegebene Bedeutung hat und

$R^8$, $R^9$ Alkoxy wie unter $R^1$ genannt,

Alkylamino wie unter $R^7$ genannt,

Dialkylamino wie unter $R^7$ genannt,

Aryloxy wie unter Y genannt, oder
Halogenalkoxy wie unter $R^1$ genannt,
bedeuten oder
$R^4$, $R^5$ gemeinsam $=CHR^6$ wie vorstehend bei $R^1$, $R^2$ definiert oder
-CO-Z-CO- wobei
Z Alkylen wie Ethyl, Propylen und Butylen,
Ethylen, welches Teil eines Cycloalkylesters wie Cyclopentyl und Cyclohexyl ist, wobei dieses Ringsystem zusätzlich ein bis vier der unter $R^1$ genannten Halogenatome, insbesondere Fluor und Chlor und/oder Alkylgruppen wie insbesondere Methyl tragen kann,
Ethylen, welches Teil eines der unter $R^6$ genannten substituierten oder unsubstituierten Aryl- oder Hetarylrings sein kann,
bedeutet.

Die erfindungsgemäßen Verbindungen I können beispielsweise mit anorganischen und organischen Säuren oder mit Alkylhalogeniden Additionssalze bilden oder sie können, sofern einer der Substituenten saure Eigenschaften hat, mit anorganischen und organischen Basen zu Salzen umgesetzt werden. Die entsprechenden Salze gehören ebenfalls zur Erfindung.

Nicht zu den erfindungsgemäßen (3-Chlor-2-methylphenyl)ethylaminen I gehören Verbindungen I in denen $R^3$ Wasserstoff bedeutet und $R^2$ nicht Wasserstoff bedeutet.

Die (3-Chlor-2-methylphenyl)ethylamine I bzw. die sie enthaltenden herbiziden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungformen richten sich nach den Verwendungszwecken; sie sollten in jedem Fall möglichst die feinste Verteilung der erfindungsgemäßen Wirkstoffe gewährleisten.

Die Verbindungen I eignen sich allgemein zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe,
z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel, wie N,N-Dimethylformamid, Dimethylsulfoxid, N-Methylpyrrolidon oder Wasser.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin-und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten zwischen 0,1 und 95 Gew.%, vorzugsweise zwischen 0,5 und 90 Gew.%, Wirkstoff. Die Wirkstoffe werden dabei in einer Reinheit von 95 % bis 100 %, vorzugsweise 97 % bis 100 % (nach NMR-Spektrum) eingesetzt.

Die erfindungsgemäßen Verbindungen I können beispielsweise wie folgt formuliert werden:

I. Man vermischt 90 Gewichtsteile der Verbindung Nr. 10 mit 10 Gewichtsteilen N-Methyl-α-pyrrolidon und erhält eine Lösung, die zur Anwendung in Form kleinster Tropfen geeignet ist.

II. 20 Gewichtsteile der Verbindung Nr. 17 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.

III. 20 Gewichtsteile der Verbindung Nr. 27 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

IV. 20 Gewichtsteile des Wirkstoffs Nr. 39 werden in einer Mischung gelöst, die aus 25 Gewichtsteilen Cyclohexanon, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

V. 20 Gewichtsteile des Wirkstoffs Nr. 51 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kie selsäuregel gut vermischt und in einer Hammermühle vermahlen. Durch feines Verteilen der Mischung in 20 000 Gewichtsteilen Wasser erhält man eine Spritzbrühe, die 0,1 Gew.% des Wirkstoffs enthält.

VI. 3 Gewichtsteile des Wirkstoffs Nr. 68 werden mit 97 Gewichtsteilen feinteiligem Kaolin vermischt. Man erhält auf diese Weise ein Stäubemittel, das 3 Gew.% des Wirkstoffs enthält.

VII. 30 Gewichtsteile des Wirkstoffs Nr. 5 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Gewichtsteile des Wirkstoffs Nr. 43 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.

Die Applikation der herbiziden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Bekämpfungsziel, Jahreszeit, Zielpflanzen und Wachstumsstadium 0,001 bis 5,0, vorzugsweise 0,05 bis 3,0 kg/ha aktive Substanz (a.S.).

In Anbetracht der Vielseitigkeit der Applikationsmethoden können die erfindungsgemäßen Verbindungen bzw. sie enthaltende Mittel noch in einer weiteren Zahl von Kulturpflanzen zur Beseitigung unerwünschter Pflanzen eingesetzt werden. In Betracht kommen beispielsweise folgende Kulturen:

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Avena sativa | Hafer |
| Beta vulgaris spp. altissima | Zuckerrübe |
| Beta vulgaris spp. rapa | Futterrübe |
| Beta vulgaris spp. esculenta | Rote Rübe |
| Brassica napus var. napus | Raps |
| Brassica napus var. napobrassica | Kohlrübe |
| Brassica napus var. rapa | Weiße Rübe |
| Brassica rapa var. silvestris | Rüben |
| Camellia sinensis | Teestrauch |
| Carthamus tinctorius | Saflor - Färberdistel |
| Carya illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cucumis melo | Melone |
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glycine max | Sojabohne |
| Gossypium hirsutum (Gossypium arboreum, Gossypium herbaceum, Gossypium vitifolium) | Baumwolle |
| Helianthus annuus | Sonnenblume |
| Helianthus tuberosus | Topinambur |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lactuca sativa | Kopfsalat |
| Lens culinaris | Linse |
| Linum usitatissimum | Faserlein |
| Lycopersicon lycopersicum | Tomate |
| Malus spp. | Apfel |

| Botanischer Name | Deutscher Name |
|---|---|
| Manihot esculenta | Maniok |
| Medicago sativa | Luzerne |
| Mentha piperita | Pfefferminze |
| Musa spp. | Obst- und Mehlbanane |
| Nicotiana tabacum (N. rustica) | Tabak |
| Olea europaea | Ölbaum |
| Oryza sativa | Reis |
| Panicum miliaceum | Rispenhirse |
| Phaseolus lunatus | Mondbohne |
| Phaseolus mungo | Erdbohne |
| Phaseolus vulgaris | Buschbohnen |
| Pennisetum glaucum | Perl- oder Rohrkolbenhirse |
| Petroselinum crispum spp. tuberosum | Wurzelpetersilie |
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sesamum indicum | Sesam |
| Solanum tuberosum | Kartoffel |
| Sorghum bicolor (s. vulgare) | Mohrenhirse |
| Sorghum dochna | Zuckerhirse |
| Spinacia oleracea | Spinat |
| Theobroma cacao | Kakaobaum |
| Trifolium pratense | Rotklee |
| Triticum aestivum | Weizen |
| Triticum durum | Hartweizen |
| Vaccinium corymbosum | Kulturheidelbeere |
| Vaccinium vitis-idaea | Preißelbeere |
| Vicia faba | Pferdebohnen |
| Vigna sinensis (V. unguiculata) | Kuhbohne |
| Vitis vinifera | Weinrebe |
| Zea mays | Mais |

Zur Verbreiterung des Wirkungsspektrums und zur Erzielung synergistischer Effekte können die (3-Chlor-2-methylphenyl)ethylamine I mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, Chinolincarbonsäurederivate, Aryloxy-, Heteroaryloxyphenoxypropionsäuren sowie deren Salze, Ester und Amide und andere in Betracht.

Außerdem kann es von Nutzen sein, die neuen Verbindungen I allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs-und Spurenelementmängeln eingesetzt werden. Es können auch nichtphytotoxische Öle und Ölkonzentrate zugesetzt werden.

Synthesebeispiele

Die in den nachstehenden Synthesebeispielen wiedergegebenen Vorschriften wurden unter entsprechender Abwandlung der Ausgangsverbindungen zur Gewinnung weiterer Verbindungen I benutzt. Die so erhaltenen Verbindungen sind in den nachstehenden Tabellen mit physikalischen Angaben aufgeführt.

Synthesebeispiele für die Benzylcyanide III

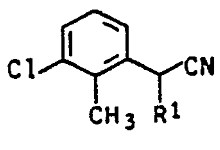

Beispiel III.1

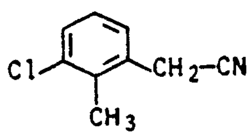

(2.4 mol) 3-Chlor-2-methylbenzylhalogenid (Bromid oder Chlorid) in 500 ml Dimethylsulfoxid wurden bei Raumtemperatur innerhalb 1 h zu einer Mischung von 156.4 g (3.2 mol) Natriumcyanid und 3500 ml Dimethylsulfoxid getropft und 5 h bei 100 °C gerührt. Nach dem Abkühlen goß man auf 15 l Wasser und rührte 2 h bei Raumtemperatur. Der Niederschlag wurde abgesaugt und mit Wasser gewaschen. Nach dem Trocknen erhielt man 320.0 g - 343.5 g (80 bis 86 %) 3-Chlor-2-methylbenzylcyanid als braune Kristalle mit Fp. 50 bis 51 °C.

Beispiel III.2

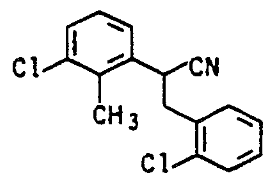

8,3 g (50 mmol) 3-Chlor-2-methylbenzylcyanid wurden bei 50 bis 60 °C zu einer Mischung aus 12,7 ml (100 mmol) 2-Chlorbenzylchlorid und 6,0 g Natronlauge in 150 ml Triethylamin gerührt. Nach 15 h Erhitzen unter Rückfluß goß man auf Wasser und extrahierte mit Ether. Nach dem Einengen des organ. Extraktes wurde der ölig-kristalline Rückstand mit wenig Ether digeriert. Ausb. 4,1 g (28 %) α-(2-Chlorbenzyl)-3-chlor-2-methylbenzylcyanid als farblose Kristalle mit Fp. 98 bis 100 °C.

Beispiel III.3

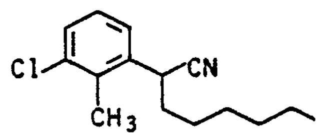

8,3 g (50 mmol) 3-Chlor-2-methylbenzylcyanid wurden bei Raumtemperatur in eine Suspension von 1,2 g (100 mmol) Natriumhydrid in 100 ml Toluol eingerührt und auf 80 °C erwärmt. Nach 30 min tropfte man 7,0 ml (50 mmol) n-Hexylbromid zu und rührte 15 h bei 80 °C. Nach dem Abkühlen auf Raumtemperatur wurde mit 100 ml Wasser hydrolysiert und die organische Phase abgetrennt. Einengen i.Vak. ergab 18,3 g (73 %) α-(n-Hexyl)-3-chlor-2-methyl-benzylcyanid als Öl.

Entsprechend den vorstehenden Beispielen wurden die folgenden Verbindungen hergestellt

| | $R^1$ | Fp |
|---|---|---|
| III.4 | $CH_2CH_3$ | öl |
| III.5 | $(CH_2)_2CH_3$ | öl |
| III.6 | $(CH_2)_5CH_3$ | öl |
| III.7 | $(CH_2)_2CH(CH_3)_2$ | öl |
| III.8 | $CH_2CH=CH_2$ | öl |
| III.9 | $CH_2C_6H_5$ | 72 °C. |

Synthesebeispiele für die Vinylcyanide V

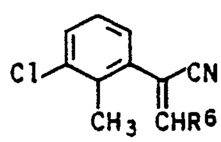

Beispiel V.1

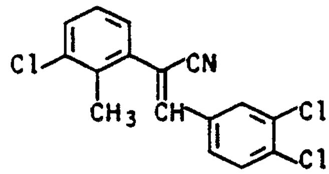

24,9 g (150 mmol) 3-Chlor-2-methylbenzylcyanid, 26,3 g (150 mmol) 3,4-Dichlorbenzaldehyd und 8,1 g (150 mmol) Natriummethylat wurden 15 h in Methanol unter Rückfluß erhitzt. Nach dem Abkühlen saugte man den Niederschlag ab. Ausbeute 26,5 g (55 %) α-(3,4-Dichlorbenzyliden)-3-chlor-2-methylbenzylcyanid als farblose Kristalle mit Fp. 128 bis 130 °C.

Unter entsprechender Abwandlung des vorstehenden Beispiels wurden die folgenden Verbindungen hergestellt.

| | $R^6$ | |
|---|---|---|
| V.2 | 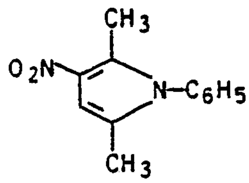 | 220°C |
| V.3 | 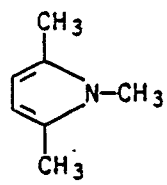 | 159-160°C |
| V.4 | $-C_6H_5$ | 108-109°C |
| V.5 | $-C_6H_4-2-OCH_3$ | 104-106°C |
| V.6 | $-C_6H_3-2-NH_2-4-NO_2$ | >240°C |
| V.7 | 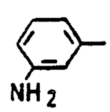 | >240°C |
| V.8 | $-C_6H_3-2-CH_3-3-Cl$ | 132-134°C |
| V.9 | $-C_6H_3-3,4-Cl_2$ | 125-128°C |
| V.10 | $-C_6H_4-2-Cl$ | 122-124°C |
| V.11 | $-C_6H_4-4-OCH_3$ | 76- 77°C |
| V.12 | $-C_6H_3-2,3-Cl_2$ | 144-145°C |
| V.13 | $-C_6H_3-2,4-Cl_2$ | 136-137'C |

Synthesebeispiele für die Nitrostyrylderivate VIII

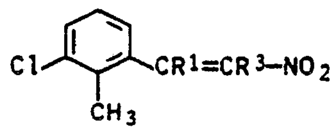

Beispiel VIII.1

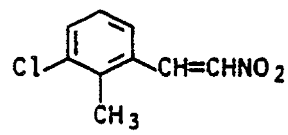

Zu einer Lösung von 7.72 g (50 mmol) 3-Chlor-2-methylbenzaldehyd und 3.35 g (55 mmol) Nitromethan in 15 ml wasserfreiem Methanol rührte man 0.90 g (5 mmol) Ethylendiammoniumdiacetat und ließ 15 h bei Raumtemperatur stehen. Nach dem Einengen i. Vak. digerierte man mit Cyclohexan und saugte ab. Ausb. 1.3 g (13 %) 3-Chlor-2-methyl-ω-nitrosyrol mit Fp. 68 bis 69 °C; aus der Mutterlauge waren durch säulenchromatographische Reinigung weitere 3.4 g (34 %) Produkt isolierbar. Die Gesamtausbeute betrug 3.7 g (47 %).

Entsprechend dem vorstehenden Beispiel wurden die folgenden Verbindungen hergestellt. Dabei wurde lediglich, abweichend vom Beispiel 15 Stunden am Rückfluß erhitzt.

| | $R^1$ | $R^3$ | | Elementaranalyse | | | | | |
|---|---|---|---|---|---|---|---|---|---|
| | | | | | C | H | N | O | Cl |
| VIII.2 | H | $CH_3$ | öl | ber.: | 56.7 | 4.7 | 6.6 | 15.1 | 16.8 |
| | | | | gef.: | 56.7 | 4.8 | 6.4 | 14.7 | 16.8 |
| VIII.3 | H | $C_2H_5$ | öl | ber.: | 58.5 | 5.3 | 6.2 | 14.2 | 15.7 |
| | | | | gef.: | 58.8 | 5.7 | 6.0 | 14.1 | 14.9 |

Synthese der Wirkstoffe

Beispiel 1

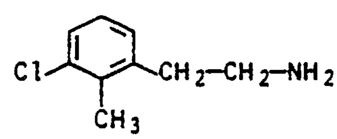

A. Synthese aus 3-Chlor-2-methylbenzylcyanid (Reduktion mit Raney-Nickel).

100 g (0.60 mol) 3-Chlor-2-methylbenzylcyanid in 200 ml Methanol wurden mit 20 g Raney-Nickel und 50 ml Ammoniak im Schüttel-Autoklaven auf 80 °C erhitzt. Durch Aufpressen von Wasserstoff stellte man einen konstanten Druck von 15 bar ein. Nach 48 h kühlte man ab, entspannte und filtrierte. Einengen des Filtrats ergab 102 g öliges Rohprodukt. Durch fraktionierte Destillation i. Vak. erhielt man 78 g (77 %) 3-Chlor-2-methylphenylethylamin mit Sdp. 74 bis 81 °C (0.4 mbar).

B. Synthese aus 3-Chlor-2-methylbenzylcyanid (Reduktion mit Lithiumaluminiumhydrid)

Zu einer Suspension von 20 g (0.52 mol) Lithiumaluminiumhydrid in 400 ml wasserfreiem Ether wurden unter Eiskühlung 25.5 g (0.26 mol) konz. Schwefelsäure innerhalb von 45 min. zugegeben. Anschließend rührte man 1 h bei Raumtemperatur; dann wurden 28 g (0.17 mol) 3-Chlor-2-methylbenzylcyanid bei Raumtemperatur so zugetropft, daß der Ether am Sieden gehalten wurde. Nach 2 h Erhitzen unter Rückfluß rührte man 15 h bei Raumtemperatur nach. Nach dem Abkühlen auf 0 bis 5 °C wurden vorsichtig 150 ml $H_2O$ (heftige Gasentwicklung) und anschließend eine Lösung von 26 g (0.65 mol) NaOH in 250 ml $H_2O$ zugetropft. Man dekantierte die organische Phase ab und digerierte den anorganischen Rückstand mit 3 mal 300 ml Ether. Die vereinigten Ether-Auszüge wurden mit $Na_2SO_4$ getrocknet und i. Vak. eingeengt. Nach fraktionierter Destillation i. Vak. erhielt man 20.5 g (72 %) 3-Chlor-2-methylphenethylamin mit Sdp. 74 bis 78 °C (0.4 mbar).

C. Synthese aus 3-Chlor-2-methyl-ω-nitrostyrol (Reduktion mit Lithiumaluminiumhydrid)

9.9 g (50 mmol) 3-Chlor-2-methyl-ω-nitrostyrol wurden bei Raumtemperatur zu einer Suspension von 3.8 g (100 mmol) Lithiumaluminiumhydrid in 100 ml trockenem Ether getropft. Nach 2 h Erhitzen unter Rückfluß kühlte man auf 5 bis 10 °C ab und versetzte tropfenweise mit 50 ml Wasser. Die organ. Phase wurde abgetrennt und der anorganische Rückstand mehrmals mit Ether digeriert. Nach dem Einengen der vereinigten und getrockneten organischen Phasen engte man i. Vak. ein und reinigte das Rohprodukt durch fraktionierte Destillation i. Vak.. Ausb.: 3.4 g (40 %) 3-Chlor-2-methylphenethylamin (Sdp. 70 bis 80 °C/0.4 mbar).

Durch Einleiten von gasförmigem HCl in die etherische Lösung des Phenethylamins erhielt man ein kristallisierbares Hydrochlorid (Fp. 210 °C/Zers.).

Beispiel 2

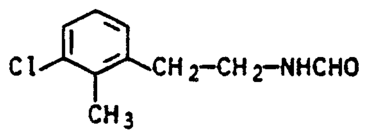

Zu 22.0 g (0.13 mol) 3-Chlor-2-methylphenethylamin tropfte man unter Rühren 5.5 ml Ameisensäure; das gebildete 3-Chlor-2-methylphenethylammoniumformiat wurde zunächst 1 h bei 50 °C und 30 Torr getrocknet und anschließend 90 min. bei 160 °C gerührt. Nach dem Abkühlen erhielt man 23.3 g (90 %) N-Formyl-3-chlor-2-methylphenethylamin mit Fp. 44 bis 46 °C.

Beispiel 3

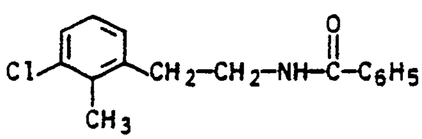

Zu einer Lösung von 25.4 g (0.15 mol) 3-Chlor-2-methylphenethylamin in 50 ml Pyridin tropfte man unter Eiskühlung 17.4 ml (0.15 mol) Benzoylchlorid. Nach 15 h Rühren bei Raumtemperatur wurde in 350 ml 4 molare Salzsäure gegossen und mit 3 mal 200 ml Ether extrahiert. Nach dem Trocknen mit $MgSO_4$ engte man i. Vak. ein und erhielt 22.7 g (55 %) N-Benzoyl-3-chlor-2-methylphenethylamin mit Fp. 129 °C.

Beispiel 4

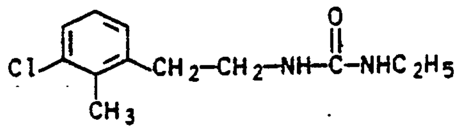

2,5 g (15 mmol) 3-Chlor-2-methylphenethylamin in 100 ml Toluol wurden bei Raumtemperatur mit 1.1 g (15 mmol) Ethylisocyanat versetzt. Nach 3d Rühren bei Raumtemperatur saugte man ab; Ausb.: 2.9 g (80 %) N-(3-Chlor-2-methylphenethyl)-N′-ethylharnstoff mit Fp. 120 °C.

Beispiel 5

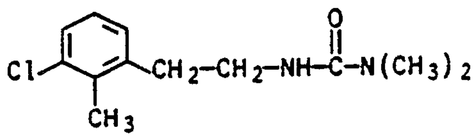

Zu einer Lösung von 2.5 g (15 mmol) 3-Chlor-2-methylphenethylamin und 2.1 ml (15 mmol) Triethylamin in 50 ml Dichlormethan tropfte man bei Raumtemperatur 1.7 g (16 mmol) Dimethylcarbamidsäurechlorid in 20 ml Dichlormethan. Nach 15-stündigem Erhitzen unter Rückfluß, kühlte man ab und wusch 3 mal mit $H_2O$. Nach dem Trocknen mit $MgSO_4$ wurde die organ. Phase eingeengt und der Rückstand mit Pentan digeriert. Ausb. 2.7 g (75 %) N-(3-Chlor-2-methylphenethyl)-N′-(dimethyl)harnstoff mit Fp. 113 bis 115 °C.

Beispiel 6

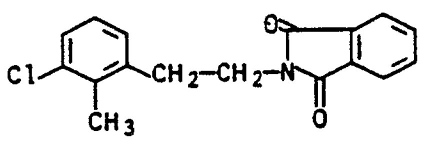

5,06 g (30 mmol) 3-Chlor-2-methylphenethylamin und 4.44 g (30 mmol) Phthalsäureanhydrid wurden in 70 ml Eisessig 3 h unter Rückfluß erhitzt. Nach dem Abkühlen auf Raumtemperatur goß man in 300 ml $H_2O$ ein und saugte den Niederschlag ab. Ausb.: 6.9 g N-(3-Chlor-2-methylphenethyl)phthalsäureimid.

Beispiel 60

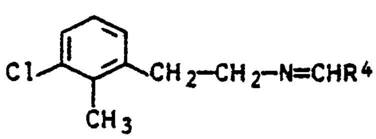

$R^4 = C_6H_3\text{-}2,4\text{-}Cl_2$

8,5 g (50 mmol) 3-Chlor-2-methylphenethylamin und 8,8 g (50 mmol) 2,4-Dichlorbenzaldehyd wurden in 70 ml Toluol 15 h am Wasserabscheider unter Rückfluß erhitzt. Nach dem Einengen i. Vak. erhielt man 13,6 g (83 %) N-(2,4-Dichlorbenzyliden)-3-chlor-2-methylphenethylamin als gelbes Öl, das nach 5 d Stehen bei Raumtemperatur durchkristallisierte (Fp. 45 °C).

Beispiel 64

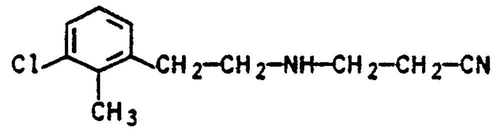

15,0 g (88 mmol) 3-Chlor-2-methylphenethylamin, 4,7 g (89 mmol) Acrylnitril und eine Spatelspitze Kupferacetat wurden 15 h unter Rückfluß erhitzt. Nach dem Abkühlen versetzte man die Reaktionsmischung mit 400 ml Wasser und extrahierte mit Dichlormethan. Der organ. Extrakt wurde eingeengt und der Rückstand chromatographisch [Aluminiumoxid, Brockmann-Aktivität 3; Dichlormethan] gereinigt. Ausbeute 8,7 g (44 %) N-(2-Cyanoethyl)-3-chlor-2-methylphenethylamin als braunes Öl.

| | | | | |
|---|---|---|---|---|
| Ber. | C 64,7 | H 6,7 | N 12,6 | Cl 16,0 |
| Gef. | C 65,0 | H 6,4 | N 13,1 | Cl 15,7 |

Beispiel 65

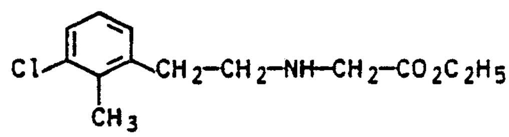

5,1 g (30 mmol) 3-Chlor-2-methylphenethylamin und 5,0 g (30 mmol) Bromessigsäureethylester wurden mit 4,2 ml (30 mmol) Triethylamin in 80 ml Toluol 15 h bei 80 °C gerührt. Nach dem Abdestillieren des Lösungsmittels reinigte man den Rückstand chromatographisch (Kieselgel/Essigester) und erhielt nach dem Abtrennen einer Vorfraktion 1,7 g (22 %) 3-Chlor-2-methylphenethylaminoessigsäureethylester als gelbliche Flüssigkeit.

$^1$H-NMR $CDCl_3$): $\delta$ = 1,27 (t, J=8Hz; 3H; $CH_2CH_3$), 1,79 (s; 1H; NH), 2,38 (s; 3H; $CH_3$), 2,80 (s; 4H, $2CH_2$), 3,42 (s; 2H; $CH_2$), 4,18 (q, J=8Hz; 2H; $CH_2CH_3$), 7,00-7,30 (m; 3H; Phenyl-H).

Beispiel 66

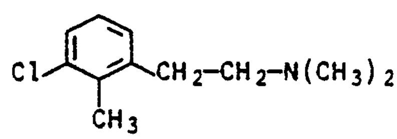

5,1 g (30 mmol) 3-Chlor-2-methylphenethylamin wurden bei 0 bis 5 °C in eine Mischung aus 5,7 ml Ameisensäure und 1 ml $H_2O$ gerührt und nach 15 min mit 5,0 ml wäßriger Formaldehydlösung (36,5 %) 15 h unter Rückfluß erhitzt. Nach 8 h Stehen bei Raumtemperatur verdünnte man mit 300 ml Wasser und extrahierte mit Dichlormethan. Nach dem Einengen des organischen Extraktes wurde der ölig-kristalline Rückstand mit wenig Cyclohexan/Propanol (3:1) digeriert; Ausbeute 0,3 g (5 %) N-Dimethyl-3-chlor-2-methylphenethylamin als farblose Kristalle mit Fp. 212 °C.

Beispiel 66 (x HJ)

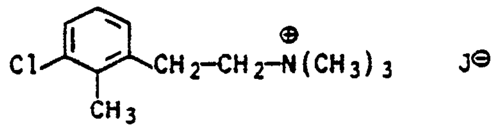

Eine Lösung von 4,2 g (25 mmol) 3-Chlor-2-methylphenethylamin in 70 ml Ethanol wurde tropfenweise mit 10,7 g (75 mmol) Methyliodid versetzt und 15 h bei Raumtemperatur gerührt. Nach dem Abdestillieren des Lösungsmittels wurde der Rückstand mit Ether digeriert und abgesaugt. Ausbeute 2,2 g (26 %) N-Trimethyl-3-chlor-2-methylphenethylammoniumiodid mit Fp. 225 bis 230 °C.

Tabelle 1

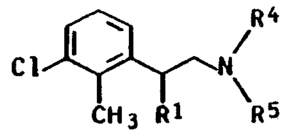

| Wirkstoff Nr. | $R^1$ | $R^4$ | $R^5$ | Fp [°C] | Kp [°C/mbar] |
|---|---|---|---|---|---|
| 1 | H | H | H | | 70-80/0,4 |
| 1 [x HCl] | H | H | H | 210 (Zers.) | |
| 2 | H | H | -CHO | 44-46 | |
| 3 | H | H | $-CO-C_6H_5$ | 129 | |
| 4 | H | H | $-CO-CH_3$ | 69-70 | |
| 5 | H | H | $-CO-C(CH_3)_3$ | 60-62 | |
| 6 | H | H | $-CO-C_6H_4-2-Cl$ | 71-73 | |
| 7 | H | H | $-CO-C_6H_3-3,4-Cl_2$ | 165 | |
| 8 | H | H | $-SO_2-C_6H_4-4-Cl$ | 74-75 | |
| 9 | H | H | $-PO[N(CH_3)_2]_2$ | öl | |
| 10 | H | H | $-PO(OC_6H_5)_2$ | 63-64 | |
| 11 | H | H | $-SO_2$ 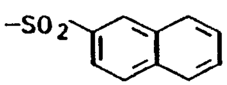 | 112-114 | |
| 12 | H | H | $-SO_2-C_6H_4-4-NO_2$ | 118-119 | |
| 13 | H | H | $-CO-CH=CH_2$ | öl | |

Tabelle 1 (Fortsetzung)

| Wirkstoff Nr. | $R^1$ | $R^4$ | $R^5$ | Fp [°C] | Kp [°C/mbar] |
|---|---|---|---|---|---|
| 14 | H | H | $-CO-C=CH_2$<br>$CH_3$ | 92-93 | |
| 15 | H | H | $-CO-CHOCOCH_3$<br>$C_6H_5$ | öl | |
| 16 | H | H | $-CO-CH_2CH_2CH_3$ | öl | |
| 17 | H | H | $-CO-CH_2C(CH_3)_3$ | öl | |
| 18 | H | H | $-SO_2-C_6H_4-4-CH_3$ | 89-91 | |
| 19 | H | H | $-SO_2-CH_3$ | 73-74 | |
| 20 | H | H | $-CO-C_6H_4-2-NO_2$ | 126-128 | |
| 21 | H | H | $-CO-C_6H_4-3-NO_2$ | 139 | |
| 22 | H | H | $-CO-C_6H_3-2,4-Cl_2$ | 86-89°C | |
| 23 | H | H | $-CO-C_6H_4-4-Cl$ | 114-115 | |
| 24 | H | H | $-CO-C_2H_5$ | 81-82 | |
| 25 | H | H | $-PS(OCH_3)_2$ | öl | |
| 26 | H | H | $-CO-C_6H_4-4-NO_2$ | 132-134 | |
| 27 | H | H | $-CO-C_6H_4-2-CF_3$ | 94 | |
| 28 | H | H | $-PO(OCH_2CCl_3)_2$ | 101-102 | |
| 29 | H | H | $-SO_2-C_6H_5$ | 92-93 | |

Tabelle 1 (Fortsetzung)

| Wirkstoff Nr. | $R^1$ | $R^4$ | $R^5$ | Fp [°C] | Kp [°C/mbar] |
|---|---|---|---|---|---|
| 30 | H | H | $-SO_2-C_6H_4-2-NO_2$ | 134-135 | |
| 31 | H | H | $-PO(OC_2H_5)_2$ | öl | |
| 32 | H | H | $-PS(OC_2H_5)_2$ | öl | |
| 33 | H | H | $-SO_2-NHCH(CH_3)_2$ | 95-98 | |
| 34 | H | H | $-CONHCH_2CH_3$ | 120 | |
| 35 | H | H | $-CS-NH-CH_3$ | 92-93 | |
| 36 | H | H | $-CO-NH-CH_2-CH_2-CH_2-CH_3$ | 101-102 | |
| 37 | H | H | $-CO-NH-C_6H_4-3-Cl$ | 117-118 | |
| 38 | H | H | $-CO-NH-C_6H_4-4-Cl$ | 200 | |
| 39 | H | H | $-CS-NH-C_6H_5$ | 124-125 | |
| 40 | H | H | $-CO-NH-C_6H_3-3,4-Cl$ | 165 | |
| 41 | H | H | $-CO-NH-CH_2-CO_2-C_2H_5$ | 108-110 | |
| 42 | H | H | -CO-NH- 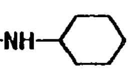 | 159-161 | |
| 43 | H | H | $-CS-CH_2-CH=CH_2$ | öl | |
| 44 | H | H | $-CON/CH_3)_2$ | 113-115 | |
| 45 | H | H | $-CO-N(C_2H_5)_2$ | öl | |
| 46 | H | H | $-CO-N(C_6H_5)_2$ | 98-99 | |

Tabelle 1 (Fortsetzung)

| Wirkstoff Nr. | $R^1$ | $R^4$ | $R^5$ | Fp [°C] | Kp [°C/mbar] |
|---|---|---|---|---|---|
| 47 | H | H | $-CO_2CH_3$ | 72-73 | |
| 48 | H | H | $-CO_2-C_2H_5$ | 54 | |
| 49 | H | H | $-CO_2-C_6H_5$ | 51-52 | |
| 50 | H | H | $-CO-N(CH_3)_2$ | 113-115 | |
| 51 | H | H | $-CO-N[CH_2-CH(CH_3)_2]_2$ | 64 | |
| 59 | H | H | $-CO(CH_2)_2CO_2H$ | 112 | |
| 64 | H | H | $-(CH_2)_2CN$ | Öl | |
| 65 | H | H | $-CH_2CO_2CH_2CH_3$ | Öl | |
| 66 | H | $CH_3$ | $CH_3$ | 212 | |
| 66 [x $CH_3$] | H | $CH_3$ | $CH_3$ | 225-230 | |
| 70 | $CH_2$-(2-$OCH_3$-Phenyl) | H | H | | |
| 70 [x HCl] | $CH_2$-(2-$OCH_3$-Phenyl) | H | H | 164-168 | |
| 71 | $CH_2$-(3-Cl, 4-Cl-Phenyl) | H | H | | |
| 71 [x HCl] | $CH_2$-(3-Cl, 4-Cl-Phenyl) | H | H | 260 (Zers.) | |
| 72 | $CH_2$-(2-Cl-Phenyl) | H | H | | |
| 72 [x HCl] | $CH_2$-(2-Cl-Phenyl) | H | H | 240 (Zers.) | |

Tabelle 1a

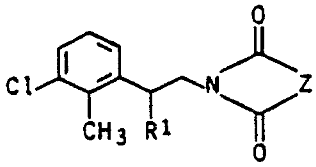

| Wirkstoff Nr. | R1 | Z | Fp [°C] |
|---|---|---|---|
| 52 | H | 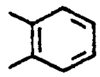 | |
| 53 | H | 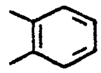 | 136 |
| 54 | H | 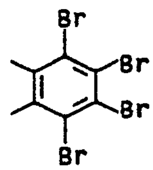 | 250 |
| 55 | H | 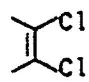 | 138 |
| 56 | H | 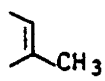 | 85 |
| 57 | H | 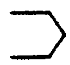 | 94 |
| 58 | H | 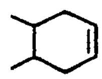 | 126 |

Tabelle 1b

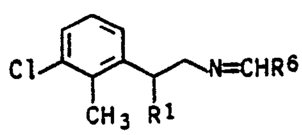

| Wirkstoff Nr. | R1 | R6 | Fp [°C] |
|---|---|---|---|
| 60 | H | 2-Cl, 4-Cl-Phenyl | 45 |
| 61 | H | -Phenyl | öl |
| 62 | H | 4-$NO_2$-Phenyl | 100-102 |
| 63 | H | 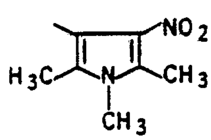 | 126 |

Tabelle 2

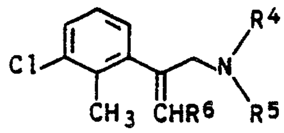

| Wirkstoff Nr. | $R^6$ | $R^4$ | $R^5$ | Fp [°C] |
|---|---|---|---|---|
| 67 | Phenyl | H | H | 230 (Zers.) |

Tabelle 3

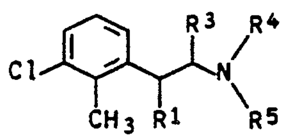

| Wirkstoff Nr. | $R^1$ | $R^3$ | $R^4$ | $R^5$ | Fp [°C] |
|---|---|---|---|---|---|
| 68 (96 alt) | H | $CH_3$ | H | H | |
| 68 [x HCl] | H | $CH_3$ | H | H | |
| 69 (97 alt) | H | $CH_2CH_3$ | H | H | |
| 69 [x HCl] | H | $CH_2CH_3$ | H | H | |

Anwendungsbeispiele

Die herbizide Wirkfung der (3-Chlor-2-methylphenylethylamine der Formel I ließ sich durch Gewächshausversuche zeigen:

Zur Aufzucht der Testpflanzen dienten Plastikblumentöpfe mit 300 $cm^3$ Inhalt und lehmigem Sand mit etwa 3 % Humus als Substrat. Die Samen der Testpflanzen wurden nach Arten getrennt flach eingesät.

Bei Vorauflaufbehandlung wurden die aufbereiteten Wirkstoffe unmittelbar danach auf die Erdoberfläche aufgebracht. Sie wurden hierbei in Wasser als Verteilungsmittel suspendiert oder emulgiert und mittels fein verteilender Düsen gespritzt. Nach dem Aufbringen der Mittel wurden die Gefäße leicht beregnet, um Keimung und Wachstum in Gang zu bringen. Danach wurden die Gefäße mit durchsichtigen Plastikhauben abgedeckt, bis die Pflanzen angewaschsen waren. Diese Abdeckung fördert ein gleichmäßiges Keimen der Testpflanzen, sofern dies nicht durch die Wirkstoffe beeinträchtigt wird. Die Aufwandmengen betrugen 2,0 kg/ha.

Zum Zwecke der Nachauflaufbehandlung wurden die Testpflanzen je nach Wuchsform erst bis zu einer Wuchshöhe von 3 bis 15 cm angezüchtet und erst dann behandelt. Die Testpflanzen wurden dafür entweder direkt gesät und in den gleichen Gefäßen aufgezogen oder sie wurden als Keimpflanzen getrennt gezüchtet und einige Tage vor der Behandlung in die Versuchsgefäße verpflanzt. Die Aufwandmengen für die Nachauflaufbehandlung betrugen 2,0 kg Wirkstoff/ha. Eine Abdeckung unterblieb bei der Nachauflaufbehandlung.

Die Versuchsgefäße wurden im Gewächshaus aufgestellt, wobei für wärmeliebende Arten wärmere

Bereiche (20 bis 35° C) und für solche gemäßigter Klimate 10 bis 25° C bevorzugt wurden. Die Versuchsperiode erstreckte sich über 2 bis 4 Wochen. Während dieser Zeit wurden die Pflanzen gepflegt und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Abkürzung | Lateinischer Name | Deutscher Name | Englischer Name |
|---|---|---|---|
| ABUTH | Abutilon theophrasti | Chinesischer Hanf | velvet leaf |
| AMARE | Amaranthus retroflexus | Zurückgekrümmter Fuchsschwanz | redroot pigweed |
| CASTO | Cassia tora | - | sicklepood |
| CHEAL | Chenopodium album | Weißer Gänsefuß | lambsquarters (goosefoot) |
| CHYCO | Chrysanthemum corinarium | Kronenwucherblume | crown daisy |
| SINAL | Sinapis alba | Weißer Senf | white mustard |
| SOLNI | Solanum nigrum | Schwarzer Nachtschatten | black nightshade |

Mit 2,0 kg/ha a.S. im Nachauflaufverfahren bzw. im Vorauflaufverfahren eingesetzt, lassen sich mit den Beispielen 3 und 24 breitblättrigew unerwünschte Pflanzen sehr gut bekämpfen.

## Ansprüche

1. (3-Chlor-2-methylphenyl)ethylamine der allgemeinen Formel I

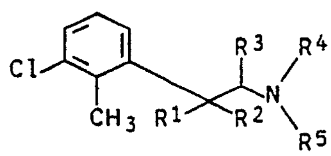

I

in der die Substituenten folgende Bedeutung haben:

$R^1$ Wasserstoff, eine $C_1$-$C_8$-Alkylgruppe, eine Arylmethylgruppe oder eine Hetarylmethylgruppe, wobei die aromatischen Reste ein bis fünf Halogenatome und/oder ein bis drei der folgenden Substituenten tragen können: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio und/oder $C_1$-$C_4$-Halogenalkylthio,

$R^2$ Wasserstoff oder gemeinsam mit $R^1$ eine Gruppe $=CHR^6$ worin

$R^6$ eine Arylgruppe oder eine Hetarylgruppe bedeutet, wobei diese aromatischen Reste ein bis fünf Halogenatome und/oder ein bis drei der folgenden Substituenten tragen können: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio und/oder $C_1$-$C_4$-Halogenalkylthio,

$R^3$ Wasserstoff, eine $C_1$-$C_6$-Alkylgruppe oder ein Arylrest, wobei der aromatische Rest ein bis fünf Halogenatome und/oder ein bis drei der folgenden Gruppen tragen kann: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio und/oder $C_1$-$C_4$-Halogenalkylthio,

$R^4$ eine der unter $R^1$ genannten Gruppen,

$R^5$ eine der unter $R^1$ genannten Gruppen,

eine $C_2$-$C_3$-Alkylgruppe, welche einen der folgenden Substituenten trägt: Nitro, Cyano, $-COR^7$ oder $SO_2R^7$ worin

$R^7$ eine Aminogruppe, eine $C_1$-$C_4$-Alkylaminogruppe, eine Di-$C_1$-$C_4$-Alkylaminogruppe, eine $C_1$-$C_4$-Alkoxygruppe oder eine der unter $R^1$ genannten Gruppen

bedeutet,

ein Rest -C(X)Y, worin

X ein Sauerstoff- oder ein Schwefelatom und

Y eine $C_1$-$C_4$-Alkylgruppe, welche einen der folgenden Reste tragen kann: Amino, $C_1$-$C_4$-Alkylamino, Di-$C_1$-$C_4$-Alkylamino, $C_1$-$C_4$-Alkoxy, Carboxyl oder $C_1$-$C_4$-Alkoxycarbonyl, eine $C_3$-$C_6$-Alkenylgruppe, welche eine Aminogruppe tragen kann, wobei diese Aminogruppe ihrerseits eine $C_1$-$C_4$-Alkoxycarbonyl-$C_1$-$C_2$-alkylgruppe und/oder ein bis zwei der unter $R^3$ genannten Gruppen tragen kann,

eine $C_1$-$C_4$-Alkoxygruppe, eine Aryloxygruppe, eine Arylaminogruppe, eine $C_1$-$C_4$-Alkylarylaminogruppe oder eine Diarylaminogruppe, eine $C_1$-$C_4$-Alkoxycarbonyl-$C_1$-$C_2$-alkylaminogruppe oder eine der unter $R^7$ genannten Gruppen
bedeutet,
$R^4$, $R^5$ gemeinsam einen Rest $=CHR^6$ wie vorstehend definiert, oder gemeinsam einen Rest

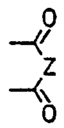 . ,

worin
Z eine $C_2$- bis $C_4$-Methylenbrücke,
eine Ethylenbrücke, welche ihrerseits Teil eines 5- bis 6-gliedrigen Cycloalkylrestes ist, wobei dieser Rest ein bis vier Halogenatome und/oder $C_1$-$C_4$-Alkylgruppen tragen kann, eine Ethenylenbrücke, welche Teil eines 5- bis 8-gliedrigen Cycloalkenylrings oder einen 5- bis 6-gliedrigen aromatischen oder heteroaromatischen Ringes sein kann, wobei diese Reste ihrerseits ein bis vier Halogenatome und/oder ein bis zwei der folgenden Gruppen tragen können: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Akylthio und/oder $C_1$-$C_4$-Halogenalkylthio
bedeutet,
wobei $R^3$ Wasserstoff bedeutet, wenn $R^2$ nicht Wasserstoff bedeutet, sowie deren umweltverträgliche Salze.

2. Verfahren zur Herstellung der Verbindungen I gemäß Anspruch 1, in denen $R^2$, $R^3$, $R^4$ und $R^5$ Wasserstoff bedeuten, dadurch gekennzeichnet, daß man ein entsprechendes Benzylhalogenid II

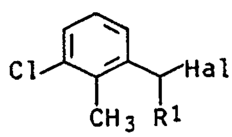 II

worin Hal ein Halogenatom bedeutet in einem inerten aprotischen organischen Lösungsmittel in Gegenwart eines Katalysators mit einem Alkalimetall- oder Erdalkalimetallcyanid zum entsprechenden Benzylcyanid III

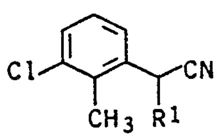 III

umsetzt und dieses anschließend in an sich bekannter Weise zum (3-Chlor-2-methylphenyl)ethylamind I ($R^2 = R^3 = R^4 = R^5 = H$)

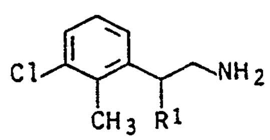 Ia

reduziert.

3. Verfahren zur Herstellung der Verbindungen I gemäß Anspruch 1, in dene $R^3$, $R^4$ und $R^5$ Wasserstoff bedeuten, dadurch gekennzeichnet, daß man ein Benzylcyanid IIIa gemäß Anspruch 2 in dem $R^1$ Wasserstoff bedeutet,

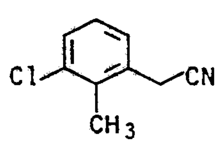 IIIa

in an sich bekannter Weise in einem inerten organischen Lösungsmittel in Gegenwart einer Base mit einem Aldehyd der Formel IV
$OCHR^6$ IV
kondensiert und das so erhaltene Vinylcyanid V

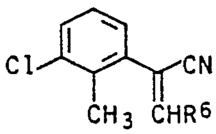
VI

anschließend in an sich bekannter Weise zum (3-Chlor-2-methylphenyl)ethylamin Ib ($R^3 = R^4 = R^5 = H$)

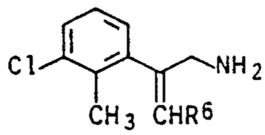
Ib

reduziert.

4. Verfahren zur Herstellung der Verbindungen I gemäß Anspruch 1, in denen $R^2$, $R^4$ und $R^5$ Wasserstoff bedeuten, dadurch gekennzeichnet, daß man ein entsprechendes Phenylcarbonylderivat VI

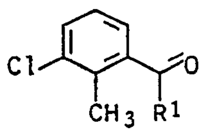
VI

in an sich bekannter Weise in einem inerten organischen Lösungsmittel in Gegenwart einer Base mit einem Nitromethanderivat VII
$R^3\text{-}CH_2NO_2$ VII zum Nitrostyrylderivat VIII

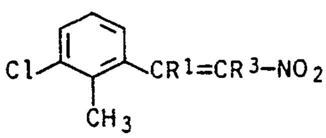
VIII

kondensiert und dieses anschließend in üblicher Weise zum (3-Chlor-2-methylphenyl)ethylamin Ic ($R^2 = R^4 = R^5 = H$)

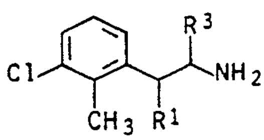
Ic

reduziert.

5. Verfahren zur Herstellung der Verbindungen I gemäß Anspruch 1, in denen $R^4$ und/oder $R^5$ nicht Wasserstoff bedeuten, dadurch gekennzeichnet, daß man ein (3-Chlor-2-methylphenyl)ethylamin Id

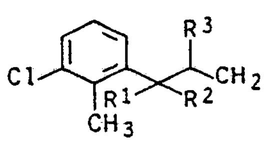
Id

in an sich bekannter Weise in einem inerten organischen Lösungsmittel in Gegenwart einer Base mit einem Halogenid der Formeln IXa bzw. IXb

| | | | | |
|---|---|---|---|---|
| $R^5$-Hal | IXa | bzw. | $R^4$-Hal | IXb |

derivatisiert.

6. Verfahren zur Herstellung der Verbindungen I gemäß Anspruch 1, in denen $R^4$ und $R^5$ gemeinsam eine Gruppe -CO-Z-CO- bedeuten, dadurch gekennzeichnet, daß man ein entsprechendes (3-Chlor-2-methylphenyl)ethylamid Id gemäß Anspruch 5 in an sich bekannter Weise in einem inerten organischen Lösungsmittel mit einem Carbonsäureanhydrid der Formel X

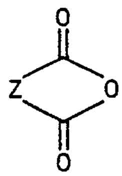

X

kondensiert.

7. Verfahren zur Herstellung der Verbindungen I gemäß Anspruch 1, in denen $R^4$ und $R^5$ gemeinsam eine Gruppe $=CHR^6$ bedeuten, dadurch gekennzeichnet, daß man ein entsprechendes (3-Chlor-2-methylphenyl)ethylamid Id gemäß Anspruch 5 in an sich bekannter Weise in einem inerten organischen Lösungsmittel in Gegenwart einer Base mit einem Aldehyd der Formel IV gemäß Anspruch 3 kondensiert.

8. Verfahren zur Herstellung der Verbindungen I gemäß Anspruch 1, in denen $R^4$ und/oder $R^5$ eine Gruppe -C(X)Y bedeuten, dadurch gekennzeichnet, daß man ein entsprechendes (3-Chlor-2-methylphenyl)-ethylamid Id gemäß Anspruch 5 in an sich bekannter Weise in einem inerten organischen Lösungsmittel in Gegenwart einer Base in ein Alken der Formel XI

(Dehydro-$R^6$) = C = X

worin (Dehydro-$R^6$) eine Gruppe $R^6$ bedeutet, welche am Bindungsatom dehydriert ist, addiert.

9. Verwendung der (3-Chlor-2-methylphenyl)ethylamine der Formel I gemäß Anspruch 1 als Herbizide.

10. Herbizide Mittel, enthaltend ein (3-Chlor-2-methylphenyl)ethylamine der Formel I gemäß Anspruch 1 und inerte Zusatzstoffe.

11. Herbizide Mittel gemäß Anspruch 10, enthaltend ein (3-Chlor-2-methylphenyl)ethylamine der Formel I und weitere wirksame Bestandteile.

12. Verfahren zur Bekämpfung unerwünschten Pflanzenwuchses, dadurch gekennzeichnet, daß man die unerwünschten Pflanzen und/oder ihren Lebensraum mit einer herbizid wirksamen Menge eines (3-Chlor-2-methylphenyl)ethylamine I gemäß Anspruch 1 behandelt.

Europäisches Patentamt

# EUROPÄISCHER RECHERCHENBERICHT

Nummer der Anmeldung

EP 89107885.9

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | BE - A - 760 968 (PRB) * Ansprüche 1,5-7; Beispiele 2-9 * -- | 1,10-12 | C 07 C 87/28<br>C 07 C 87/29<br>C 07 C 103/375<br>C 07 C 103/76<br>C 07 C 103/58 |
| A | FR - A - 1 362 148 (CIBA) * Beispiel 1 * ---- | 1 | C 07 C 143/78<br>C 07 C 127/17<br>C 07 C 127/19<br>C 07 C 125/063<br>C 07 C 121/42<br>C 07 C 101/04<br>C 07 C 119/10<br>C 07 C 157/02<br>C 07 D 209/48<br>C 07 D 207/34<br>C 07 D 207/448<br>C 07 F 9/22<br>A 01 N 33/04<br>A 01 N 37/18 |
| | | | RECHERCHIERTE SACHGEBIETE (Int. Cl.4) |
| | | | C 07 C 87/00<br>C 07 C 125/00<br>C 07 C 127/00<br>C 07 C 103/00 |

Der vorliegende Recherchenbericht wurde für alle Patentansprüche erstellt.

| Recherchenort | Abschlußdatum der Recherche | Prüfer |
|---|---|---|
| WIEN | 02-08-1989 | KÖRBER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

Europäisches Patentamt

# EUROPÄISCHER RECHERCHENBERICHT

Nummer der Anmeldung

-2-
EP 89107885.9

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| | | | A 01 N 41/06<br>A 01 N 43/36<br>A 01 N 47/28<br>A 01 N 47/10<br>A 01 N 57/26 |
| | | | RECHERCHIERTE SACHGEBIETE (Int. Cl.4) |
| | | | |

Der vorliegende Recherchenbericht wurde für alle Patentansprüche erstellt.

| Recherchenort | Abschlußdatum der Recherche | Prüfer |
|---|---|---|
| WIEN | 02-08-1989 | KÖRBER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument